# EUROPEAN PATENT SPECIFICATION

(11) **EP 1 410 015 B1**
(45) Date of publication and mention of the grant of the patent: **24.01.2007**
(21) Application number: 02754780.1
(22) Date of filing: 27.06.2002
(51) Int. Cl.: G01N 33/487

(54) **METHOD AND CIRCUITRY FOR CONTROLLING AND MEASURING ELECTRICAL PARAMETERS IN A BIOLOGICAL MEMBRANE**
VERFAHREN UND SCHALTUNG ZUR KONTROLLE UND MESSUNG ELEKTRISCHER PARAMETER IN EINER BIOLOGISCHEN MEMBRAN
PROCEDE ET CIRCUITS DE COMMANDE ET DE MESURE DE PARAMETRES ELECTRIQUES DANS UNE MEMBRANE BIOLOGIQUE

(30) Priority: 27.06.2001 EP 01115427
(43) Date of publication of application: 21.04.2004
(73) Proprietor: flyion GmbH, 72076 Tübingen (DE)
(72) Inventor: LEPPLE-WIENHUES, Albrecht, 72070 Tübingen (DE)
(74) Representative: Patentanwälte Ruff, Wilhelm, Beier, Dauster & Partner
(86) International application number: PCT/EP2002/007123
(87) International publication number: WO 2003/003005

(56) References cited:
- US-A- 6 048 722
- US-A- 6 163 719
- F.J. SIGWORTH: "Design of the EPC-9, a computer-controlled patch-clamp amplifier. 1. Hardware" JOURNAL OF NEUROSCIENCE METHODS., vol. 56, 1995, pages 195-202, XP001052696 ELSEVIER SCIENCE PUBLISHER B.V., AMSTERDAM., NL ISSN: 0165-0270 cited in the application
- DATABASE BIOSIS [Online] BIOSCIENCES INFORMATION SERVICE, PHILADELPHIA, PA, US; GILLIS KEVIN D: "Admittance-based measurement of membrane capacitance using the EPC-9 patch-clamp amplifier" Database accession no. PREV200000168696 XP002187524 & PFLUEGERS ARCHIV EUROPEAN JOURNAL OF PHYSIOLOGY, vol. 439, no. 5, March 2000 (2000-03), pages 655-664,
- HAMILL O P ET AL: "IMPROVED PATCH-CLAMP TECHNIQUES FOR HIGH-RESOLUTION CURRENT RECORDING FROM CELLS AND CELL-FREE MEMBRANE PATCHES" PFLUEGERS ARCHIV, SPRINGER VERLAG, BERLIN, DE, vol. 391, 1981, pages 85-100, XP000196663 ISSN: 0031-6768
- HUA-GUANG KANG ET AL: "Low Noise Design Of Patch Clamp Amplifier" ENGINEERING IN MEDICINE AND BIOLOGY SOCIETY, 1990., PROCEEDINGS OF THE TWELFTH ANNUAL INTERNATIONAL CONFERENCE OF THE IEEE PHILADELPHIA, PA, USA 1-4 NOV. 1990, NEW YORK, NY, USA,IEEE, US, 1 November 1990 (1990-11-01), pages 1103-1104, XP010035796 ISBN: 0-87942-559-8
- F. CELENTANO, A. GAFFURI: "Electronic clamp device for biological investigations" JOURNAL OF PHYSICS E. SCIENTIFIC INSTRUMENTS., vol. 13, no. 11, November 1980 (1980-11), pages 1219-1221, XP002187523 IOP PUBLISHING, BRISTOL., GB ISSN: 0022-3735

## Description

The invention relates to a method as well as a circuitry for controlling and measuring electrical parameters through a biological membrane. Within the context of this specification the word "comprises" is taken to mean "includes" and is not intended to mean "is limited to only".

Electronic circuitry is used for measuring and controlling electrical parameters required in order to study and manipulate the function of membrane proteins (e.g. ion channels and carriers) that carry charged particles (e.g. ions) across the lipid membranes surrounding cells. Many pharmacological compounds act via modulation of ion channels, but the development of novel agents is hindered considerably by the difficulty of measuring at high troughput function and modulation of function.

Typical methods to study such proteins are the patch-clamp technique and the lipid bilayer technique. For a description and an exemplary method of the patch clamp technique see European Patent Application file No. 00116515.8. The term "biological membrane" used herein includes cellular outer and inner membranes, membanes of subcellular organelles, artificial membranes such as lipid bilayers and other membranes known to a person skilled in the art. The term "membrane proteins" includes ion channels, other channels, transporters, receptors and proteins partially or completely embedded in the membrane or associated to the membrane.

For a typical measurement of electrical parameters in membrane proteins and membranes a device containing a metal electrode (e.g. a pipette) is connected to a lipid membrane surrounding a cell or another biological or artificial structure and the electrode is electrically coupled to an analog amplifier. That amplifier performs a sensitive current-to-voltage conversion at low noise and controls the voltage across the membrane in variable patterns. The amplifier typically contains a variety of analog electrical circuits consisting of operational amplifiers, capacitors and resistors.

Several artifacts typically disturb the measurement of electrical parameters in said membrane structures.

Because of the small geometric dimensions of biological membranes and the measuring devices connected to them, the capacitive charge movements can exceed the currents under observation. Therefore, special attention has been assigned to compensate for and cancel capacitive current transients resulting from changes in command voltage. Another important problem is the series access resistance between the membrane structure of interest and the recording electrode introducing voltage and current errors (e.g. in patch-clamp experiments in the whole-cell configuration).

The typical measuring amplifier employs three different circuitries to compensate for
1. "fast" capacitance (resulting mainly from the measuring chamber, e.g. the patch pipette or from small membrane areas)
2. "slow" capacitance (resulting mainly from larger biological membrane areas, e.g. "whole cell recordings") in series with a series resistance in the order of several MegaOhms
3. series resistance (resulting from a narrow or limited access to the membrane)

These circuitries inject currents into the measuring probe through capacitors and change the command voltage time course in order to compensate for capacitive charge movements and distortions of the desired signal.

The analog circuitry allows either "voltage clamp" controlling a constant or variable voltage while measuring the current or "current clamp" controlling the current at a desired constant value or time pattern and measuring the voltage. Modern analog circuitry introduces very low noise levels mainly stemming from capacitive and thermal fluctuations, typically in the range of 100 fA (10-3000 Hz) RMS.

The prior art in the form of Gillis, Kevin D: "Admittance-based measurement of membrane capacitance using the EPC-9 patch-clamp amplifier" and F.J. Sigworth: "Design of the EPC-9, a computer-controlled patch-clamp amplifier. 1. Hardware" describes the use of the EPC-9 amplifier. As can be taken from figure 5 as well as figure 7 of the article of Gillis, Kevin D, a cascade of switches, capacitors, resistors and amplifiers is used to build up a resistance/capacitance value that shall represent the compensation signals.

Major problems with the analog amplifier compensation circuitry are
- miniaturization for measurements in parallel channels is hindered by the large number of operational amplifiers and passive electronic elements required
- capacitive coupling, capacitive noise and crosstalk between the channels severely limits miniaturization and arrangement of multiple devices and circuits
- by design (with the use of single analog RC circuitry) only single RC combinations can be compensated
- only a limited range of RC values can be compensated

The present invention addresses the problems associated with the known measuring methods and measuring circuitry. The principal objectives of the present invention are;
- Miniaturization of the amplifier design by reducing the number of active and passive analog circuitry elements for parallel measurements in biological membranes using multiple electrodes
- Reduction of capacitive coupling, noise and crosstalk between electrodes by reducing the number of active and passive analog circuitry elements
- Compensation of complex combinations of capacitances and resistances frequently encountered when measuring electrical parameters in biological membranes
- Extending the range of capacitances / resistances that can be compensated

These problems are solved by a method with the features of claim 1 as well as by an electronic circuitry with the features of claim 21. Several embodiments of the invention are included in the subclaims and are described below. The wording of the claims is included in the description by explicit reference.

The invention describes a method and a digital electronic amplifier designed to measure and control in biological membranes voltages between -500 and +500 mV and currents ranging from a few hundred femtoamperes to hundreds of nanoamperes.

The advantages offered by the present invention are achieved by the replacement of conventional analog circuitry for correction of the frequency/transient response of the system. With the present invention this correction is performed instead by software on a computer, that in one embodiment reads current responses from the output of an operational amplifier via an AD converter and controls the voltage applied to the probe via a DA converter connected to another operational amplifier.

The software is designed to control the system in such a way that a preferred current response pattern, e.g. a square step, can be achieved by applying a complex voltage pattern that includes the required charging transients and time constants for a given stray capacitance/resistance combination. The software is also designed to estimate electrical parameters like stray capacitances, series resistances and combinations thereof by stepwise or continuous approximation and to optimize the preferred current response pattern.

With digital compensation of the capacitances and series resistances of the measuring circuitry performed by a computer connected to the measuring circuitry via AD/DA-converters it is possible to fulfill the following requirements:
- Miniaturization by reducing the number of active and passive analog circuitry elements
- Reduction of capacitive coupling, noise and crosstalk between channels
- Low electrical noise (typically 100 fA rms 10-3000 Hz)
- Simple gain switching (1-100 mV/pA)
- Simple compensation of a large range of capacitances and resistances
- Compensation of complex combinations of capacitances and resistances
- Rapid and exact forcing of the desired command voltage (voltage clamp)
- Measurement and compensation of capacitive charge movement in the measuring device and the membrane
- Compensation for current and voltage errors arising from series resistances and stray capacitances in the measuring device

These features are essential for assays measuring electrical properties of biological membrane proteins whose function typically depends on voltage and timing of voltage changes. The improvements achieved with the current invention are especially important for multichannel design of electrophysiological assays to perform multiple measurements in biological membranes at the same time.

In a preferred method the measuring circuitry is an I-V-converter connected to the biological membrane. An output of the I-V-converter can be connected via an AD-converter to the computer for analyzing the I-output signal, wherein preferably the computer calculates a V-input signal being input via a DA-converter to the measuring circuitry.

In one embodiment the invention relates to a digital measuring system for measuring and controlling current and voltage across biological membranes. The invention uses digital compensation of capacitances and series resistances instead of analog circuitry. These improvements are preferably achieved in the invention by connecting an AD-converter (ADC) to the Imon output of the I-V-converter and a DA-converter (DAC) to the Vcom input of the I-V-converter. The ADC is connected to a computer, e.g. a personal computer. The same computer controls the DAC. The computer may generate a rectangular, trapezoidal or sinusoidal voltage pattern through the DAC. The computer samples the current response I-output signal of the biological element/electrode assembly through the ADC. The computer calculates the electrical parameters of the entire circuitry including the biological element. The computer then changes the V-input signal in a way to achieve the desired current response of the entire circuitry including the biological element. Preferably a V-input signal can be generated by the computer as a result of calculating the measuring circuitry characteristics.

Furthermore, it is possible to generate a correction voltage signal by the computer. This signal is input via a DAC and an amplifier by means of a serial combination of a resistor and capacitor. The resistor or the capacitor may be switchable, but in another embodiment they may also be non-switchable. This is the case for all capacitors in this context. Preferably the correction voltage signal is input to the electrode located near membrane.

The capacitive charge movements can typically be distinguished from currents carried by ionic fluxes by timing, voltage dependence and several other means known to a person skilled in the art.

In a preferred embodiment the I-output response of a biological membrane to a voltage change is assumed to follow Ohm's law with no ion transport proteins opened within a voltage range known to a person skilled in the art.

In a preferred embodiment both membranes of a cell are intact and are arranged serially. In this arrangement the capacitance introduced by the biological membrane is small, the resistance of the preparation is large and the digital compensation can compensate for capacitances and series resistances in the recording apparatus.

In a further preferred embodiment on emembrane of a cell is physically disrupted or rendered electrically permeable. This may be done by chemical agents, for example ionophors. In this arrangement the capacitance introduced by the biological membrane is large, for example 4-100 pF. The resistance in series with the capacitance is small, for example 2-50 Mohm. The digital compensation can compensate for capacitances and series resistances in the recording apparatus.

In another preferred embodiment one membrane of a cell may physically or electrically be disrupted. In this arrangement the capacitance introduced by the biological membrane can be large, for example 4-100 pF, while the resistance in series with the capacitance is small, for example 2-50 MOhm. The digital compensation can compensate for capacitances and series resistances in the recording apparatus.

In a preferred embodiment the I-output response of a biological membrane to a voltage change is anticipated with ion transport proteins blocked. The ion transport proteins are preferably blocked by application of a solution blocking the transport protein. The solution preferably contains substances blocking the channel, e.g. heavy metal ions or pharmaceutical compounds known to a person skilled in the art. Following calculation/compensation of the measuring circuitry characteristics the blocking agents are removed by solution exchange.

In a preferred embodiment the I-output response of a biological membrane is anticipated with permeable ions removed from the solutions surrounding the membrane protein or removed from one side of the protein with the voltage forced to a value that brings the elctrochemical driving force close to 0.

In a preferred embodiment the I-output response of a biological membrane is anticipated with cofactors removed from the solutions surrounding the membrane protein or removed from one side of the protein so that opening of the ion transport protein is prevented, for example Ca2+ ions or other second messengers regulating the opening of the channel protein. Such cofactors required for the opening of ion transport proteins are known to a person skilled in the art.

In one embodiment a membrane containing a voltage gated ion channel is examined. The ion channel can be closed for calculation of the measuring circuitry characteristics by avoiding voltages in the V-input signal that open the channel, e.g. by maintaining the membrane hyperpolarized, or by keeping the V-input signal at levels that inactivate the channel, e.g. by maintaining a depolarizing voltage. The voltage patterns, solutions and reagents required for such maneuvers with voltage-dependent ion transport proteins or other ion transport proteins are known to a person skilled in the art.

It is furthermore possible to measure the electric properties of a cell membrane or a ion transport protein in said membrane, wherein voltage-dependent membrane proteins are examined. The membrane proteins may be either inactivated or activated for calculation of the measuring circuitry characteristics. The calculation can take place by choosing voltage sequences in the V-input signal that inactivate or activate the membrane proteins.

In a preferred embodiment the V-input signal is optimized for easy detection or evaluation, for example a rectangular, trapezoidal or sinusoidal signal.

In a preferred embodiment the V-input signal and the I-output signal are compared in order to calculate changes over time in membrane capacitance, for example by changes in membrane area through membrane fusion or membrane retrieval. Preferably the V-input signal is sinusoidal and the phase shift between input- and output signals is calculated by the software.

In a preferred embodiment the digitally calculated correction voltage signals may be input into the measuring circuitry for compensating capacitive currents caused by the membrane or the recording apparatus, respectively. The capacitances can be stray capacitances.

In a preferred embodiment a correction voltage signal can be generated by the computer and input via the DAC by other connective elements like amplifiers or filters by means of a variable resistor and capacitor. The correction voltage signal will preferably be fed into the electrode contacting the solution surrounding the membrane.

With the invention the following measurements and compensations can be performed:
- Measurement and compensation of the capacitance of the recording device that holds the membrane, e.g. capacitance of a pipette or a flat perforated chip
- Measurement and compensation of capacitance of the biological membrane
- Measurement and compensation of capacitance of portions of the biological membrane
- Measurement and compensation of series resistance to the biological membrane
- Measurement and compensation of combinations of series resistances and capacitances for portions of the biological membrane.

Measurements can be replaced by approximations if a partial compensation is sufficient in order to measure the properties of the ion transport protein embedded in the membrane.

The invention allows analysis of electrical signals, analysis of the electrical characteristics of the measuring circuitry and the device holding the membrane, and analysis of electrical parameters of the membrane and embedded proteins. With the invention time and frequency responses of the measuring circuitry including the membrane can be varied and analyzed. With the invention complex arrangements of capacitive and resistive elements can be analyzed and compensated.

With the invention electronic circuitry can be miniaturized and automated in order to perform multiple parallel electrical measurements in biological membranes. With the invention said analysis and compensation can be automated.

These and further features can be gathered from the claims, description and drawing and the individual features, both single and in combinations, can be implemented in an embodiment of the invention and in other fields and can represent advantageous, independently protectable constructions for which protection is claimed here. The subdivision of the application into individual sections and the subtitles in no way restrict the general validity of the statements made thereunder.

### Brief description of the drawings

Embodiments of the invention are described in greater detail hereafter relative to the following drawings:
- Fig. 1: shows a sketch of a probe containing a biological membrane, an I-V-converter, AD/DA-converters and a computer; and
- Fig. 2: shows a variation of the I-V-converter in Fig. 1 with an additional DA-converter and amplifier.

### Detailed description of the embodiments

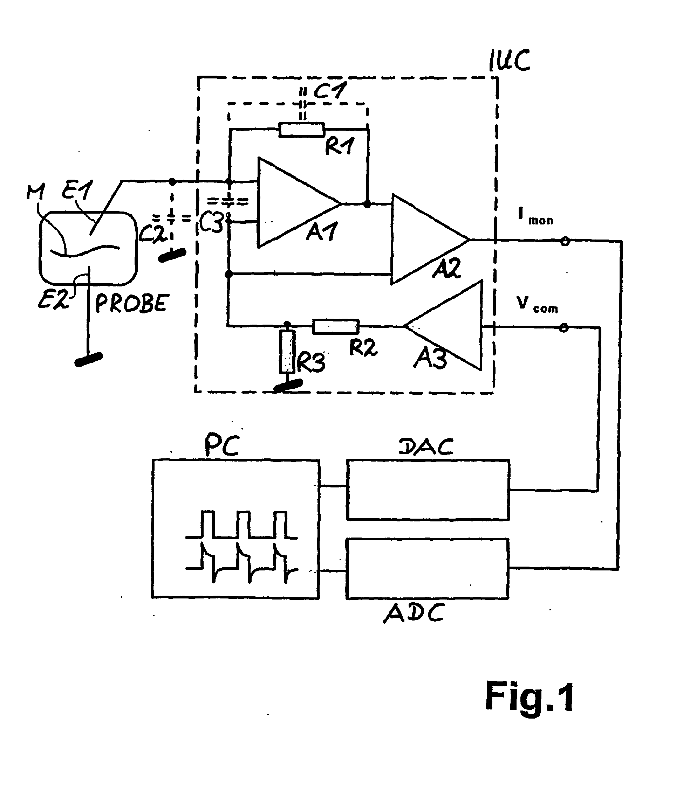

The digital measuring system illustrated in FIG. 1 comprises a measuring probe PROBE, preferably consisting of a biological membrane M in aqueous solution and two electrodes E1 and E2, preferably silver-silver/chloride electrodes. The biological membrane is placed in an electrolytic solution which is connected to system ground through E2 as reference electrode. The biological membrane is located in a chamber designed to provide high resistance parallel to that membrane, e.g. a patch-clamp pipette or a chamber for lipid bilayer techniques, see above European Patent Application Nr. 00 11 6515.8.

The electrode E1 is electrically connected to one input of an operational amplifier Al incorporated in a current-to-voltage converter circuitry IVC. The converter IVC includes a very high resistance feedback circuitry comprising a precision resistor R1 connecting the output of amplifier A1 back to the electrode E1. The other input to amplifier A1 is connected to a source of reference potential comprising an amplifier A3. The output of amplifier A3 is connected to the input of amplifier A1 through a series resistor R2, which is connected to system ground by a shunt resistor R3. The output of the reference voltage amplifier A3 is also connected to the shielding of the converter.

The output of the operational amplifier A1 in the current-to-voltage converter IVC is connected to an input of another differential amplifier A2. The second input to amplifier A2 is derived from the reference amplifier A3. The output of amplifier A2 represents the current monitor signal (Imon), representing the output signal, and is connected to an AD converter ADC. The digital output of the AD converter ADC is connected to a computer PC, in this case being a personal computer.

The input of the reference source amplifier A3 represents the command voltage (Vcom), representing the input signal. The input may comprise an operational amplifier and is connected to the analog output of a DA converter DAC, which is digitally controlled by the computer PC. A3 serves to apply a command voltage to the probe, for example a voltage step for the activation of an ionic channel in the biological membrane M.

In the circuitry illustrated in FIG. 1, the precision feedback resistor R1 may have a resistance ranging from the order of ten gigaohms to only several megaohms depending on the current range to be expected. Preferably, resistor R1 is a colloid film resistor with low capacitance selected for low noise, though other types may be used. Amplifier A1 may be a LF356 (National Semiconductor). Amplifier A1 may also consists of a preamplifier stage using a 0430 dual junction field-effect transistor (JFET), followed by a conventional integrated-circuit op-amp. The JFETs are characterized by low voltage noise and input capacitance and have small gate leakage currents. Amplifiers A2 and A3 may be Philips type NE 5534 or National Semiconductor type LF 356 selected for low voltage noise, especially above one kHz.

Distributed or "stray" capacitances are important in the operation of the system. One of these is the distributed feedback capacitance C1, which is of the order of 0.1 pF. The stray capacitance C2 between the electrode connection and ground depends on the probe containing the biological membrane and is in the range of 4-10 pF. C3 represents the input capacitance of amplifier A1 at approximately 4 pF as indicated above. A complex array of capacitances and resistances can occur in the PROBE depending on the area of the biological membrane, the geometry and material surrounding membrane and PROBE etc.

The improvement provided by the present invention comprises the replacement of conventional analog circuitry for correction of the frequency/transient response of the system. This correction is performed by a software on a computer PC, that reads current responses from the output of amplifier A1 via the AD converter ADC and controls the voltage applied to the probe via the DA converter DAC connected to amplifier A3. The software on computer PC is designed to control the system in such a way that a preferred current response pattern, e.g. a square step, can be achieved by applying a complex voltage pattern that includes the required charging currents and time constants for given stray capacitance/resistance combinations.

For comparison a typical state-of-the-art analog circuitry is described in "Design of the EPC-9, a computer controlled patch-clamp amplifier" by Sigworth, Journal of Neuroscience Methods 56 (1995), 195 - 202.

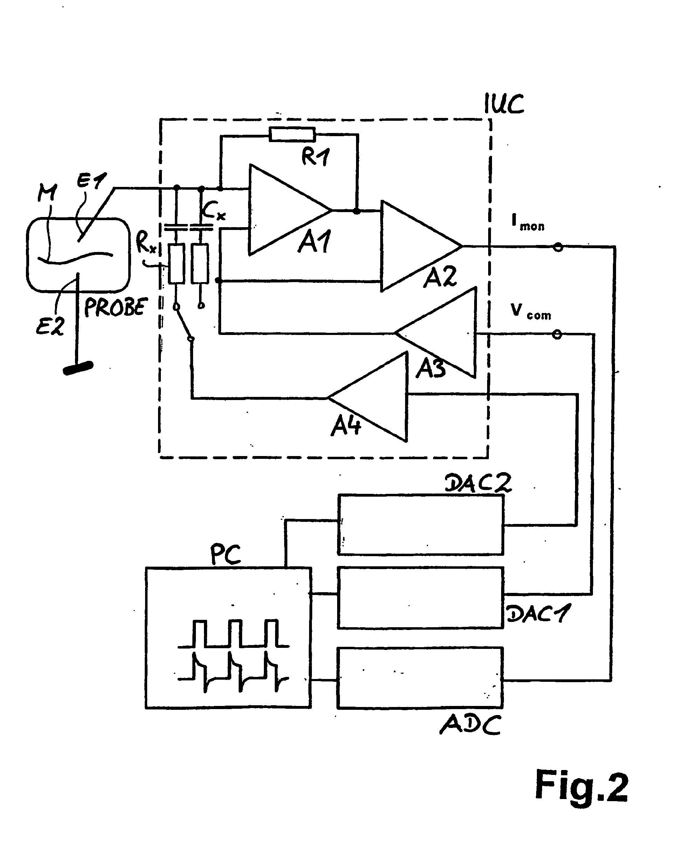

In Fig.2 another preferred embodiment of the digital measuring system is shown. The output of a second DAC2 is connected to the input of amplifier A4 via one or several different variable and exchangeable capacitors CX in series with resistors RX, e.g. 1 and 10 pF and 1 and 10 MOhm. To keep the input current within the measuring range, currents scaled by the software and put out via DAC2 can be injected through amplifier A4 to cancel capacitive currents for the charging of the pipette or chamber, biological membrane M, and stray capacitances.

### Description of a typical experiment:

A biological membrane, e.g. a cell membrane of a CHO cell expressing a transport protein, e.g. a voltage gated Na⁺ channel, is sealed to a chamber, e.g. a patch-clamp pipette in aqueous salt solution. The command voltage Vcom is continuously held "hyperpolarized" at -100 mV in order to avoid activation of the Na+ channel. Voltage steps to -105 and -95 mV of 10 ms duration are applied alternatively every 20 ms via DAC in Fig. 1. Due to stray capacitance and resistance combinations in the biological preparation the rectangular steps in the command voltage evoke overshooting, exponentially declining current spikes. These spikes are digitally read by the computer PC and the command voltage steps are distorted in order to "overcharge" the membrane M, that is a repetitive change of command voltage curves in order to approximate the current response to a rectangular response is performed by the software. Following approximation the computer PC can calculate the complex chain of resistors and capacitances and "cancel" out the charging currents by distortion of the voltage command step.

Next a command voltage step from a holding potential of -90 mV to 40 mV is applied, opening the voltage-gated Na⁺ channel. The distortions of the command voltage are now scaled up to the magnitude of the voltage step. The current through the channel is now measured without charging currents and series resistance current/voltage errors.

In this example, opening of the Na⁺ channel is avoided during the measurement/ approximation of capacitive currents and cancellation by maintaining the membrane hyperpolarized. Alternatively, an ion transport protein can be blocked by choosing a useful aqueous solution either containing substances that block the channel or by omitting permeant molecules. Following the cancellation procedure the solution can be changed.

## Claims

1. Method for control and measurement of electrical parameters through a biological membrane (M) with a measuring circuitry, wherein capacitances and series resistances of the measuring circuitry (IVC) are compensated, **characterized in that** digital compensation is used instead of analog circuitry, wherein the digital compensation is performed by a computer (PC) connected to the measuring circuitry via AD/DA-converters (ADC, DAC, DAC1, DAC2).

2. Method according to clai 1, wherein the measuring circuitry is an I-V-converter (VC) connected to the biological membrane (M), an output of the I-V-converter being connected via an AD-converter (ADC) to the computer (PC) for analysing the I-output signal, wherein preferably the computer calculates a V-input signal being input via a DA-converter (DAC, DAC1) to the measuring circuitry.

3. Method according to claim 2, wherein a V-input signal from one of the following group of signals is generated: rectangular, trapezoidal, sinusoidal.

4. Method according to one of the preceding claims, wherein the characteristics of the measuring circuitry (IVC), preferably of the biological Membrane (M) including its connections, are calculated by the computer (PC) by processing the I-output signal as a response to a typical V-input signal.

5. Method according to one of the preceding claims, wherein a V- input signal is generated by the computer (PC), preferably as a result of calculating the measuring circuitry (IUC) characteristics, the V-input signal effecting a predetermined I-output signal from the measuring circuitry.

6. Method according to claim 5, wherein the predetermined I-output signal is optimized for being simply detected or evaluated, preferably being a rectangular signal.

7. Method according to one of the preceding claims, wherein digital correction voltage signals are input into the measuring circuitry (IUC) for compensating capacitive currents at the membrane (M) or the recording chamber or holder, respectively, the capacitances preferably being stray capacitances.

8. Method according to claim 7, wherein a correction voltage signal is generated by the computer (PC) and is input via a DA-converter (DAC, DAC1) and an amplifier (A4) by means of a variable or switchable capacitance (CX), preferably being input to the electrode located near membrane (M).

9. Method according to claim 7, wherein a correction voltage signal is generated by the computer (PC) and is input via a DA-converter (DAC, DAC1) and an amplifier (A4) by means of a serial combination of a resistor and capacitor (RX, CX), preferably being input to the electrode located near membrane (M).

10. Method according to one of the preceding claims, wherein the electric properties of a cell membrane or a ion transport protein in said membrane (M) are measured, wherein the membrane or the protein are inactivated during calculation of the measuring circuitry characteristics, the inactivation preferably being effected by maintaining the membrane hyperpolarized.

11. Method according to one of the preceding claims, wherein the electric properties of a cell membrane or a ion transport protein in said membrane (M) are measured, wherein the I-output response of a biological membrane to a voltage change is assumed to follow Ohm's law.

12. Method according to one of the preceding claims, wherein the electric properties of a cell membrane or a ion transport protein in said membrane (M) are measured, wherein both membranes of a cell are intact and are arranged serially, where the capacitance introduced by the biological membrane is small, the resistance of the preparation is large and the digital compensation can compensate for capacitances and series resistances in the recording apparatus.

13. Method according to one of the preceding claims, wherein the electric properties of a cell membrane or a ion transport protein in said membrane (M) are measured, wherein one membrane of a cell is physically or electrically disrupted, wherein in this arrangement the capacitance introduced by the biological membrane is large, the resistance in series with the capacitance is small and the digital compensation can compensate for capacitances and series resistances in the recording apparatus.

14. Method according to one of the preceding claims, wherein the electric properties of a cell membrane or a ion transport protein in said membrane (M) are measured, wherein the I-output response of a biological membrane to a voltage change is anticipated with ion transport proteins blocked, where the ion transport proteins are preferably blocked by application of a solution blocking the transport protein, where the solution preferably contains substances blocking the channel, and where following calculation/compensation of the measuring circuitry characteristics the blocking agents are removed by solution exchange.

15. Method according to one of the preceding claims, wherein the electric properties of a cell membrane or a ion transport protein in said membrane (M) are measured, wherein the I-output response of a biological membrane is anticipated with permeable ions removed from the solutions surrounding the membrane protein or removed from one side of the protein with the voltage forced to a value that brings the electrochemical driving force close to 0.

16. Method according to one of the preceding claims, wherein the electric properties of a cell membrane or a ion transport protein in said membrane (M) are measured, wherein the I-output response of a biological membrane is anticipated with cofactors removed from the solutions surrounding the membrane protein or removed from one side of the protein so that opening of the ion transport protein is prevented, for example Ca²⁺ ions or other second messengers regulating the opening of the channel protein.

17. Method according to one of the preceding claims, wherein the electric properties of a cell membrane or a ion transport protein in said membrane (M) are measured, wherein voltage-dependent membrane proteins are examined, wherein the membrane proteins are inactivated for calculation of the measuring circuitry characteristics by avoiding voltages in the V-input signal that activate the membrane proteins, e.g. by maintaining the membrane hyperpolarized, or by keeping the V-input signal at levels that inactivate the channel, e.g. by maintaining a depolarizing voltage.

18. Method according to one of the preceding claims, wherein the electric properties of a cell membrane or a ion transport protein in said membrane (M) are measured, wherein voltage-dependent membrane proteins are examined, wherein the membrane proteins are either inactivated or activated for calculation of the measuring circuitry characteristics by choosing voltage sequences in the V-input signal that inactivate or activate the membrane proteins.

19. Method according to one of the preceding claims, wherein the electric properties of a cell membrane or a ion transport protein in said membrane (M) are measured, wherein the V-input signal and the I-output signal are compared in order to calculate changes over time in membrane capacitance, for example by changes in membrane area through membrane fusion or membrane retrieval, wherein the v input signal is preferably sinusoidal and the phase shift between input- and output signals is calculated by the software.

20. Method according to one of the preceding claims, wherein the V-input signal and the I-output signal are compared in order to calculate changes over time in membrane capacitance, for example by changes in membrane area through membrane fusion or membrane retrieval. Preferably the V input signal is sinusoidal and the phase shift between input- and output signals is calculated by the software.

21. Measuring circuitry for control and measuring of electrical current through a biological membrane, comprising an I-V-converter circuitry (IVC), the I-V-converter having a connecting amplifier (A1) for connection to a probe, an input amplifier (A3) and an output amplifier (A2), wherein the input amplifier and the output amplifier are connected via DA/AD-converters (DAC, ADC) to a computer (PC), with means for compensating capacitances and series resistances, **characterized by** a digital compensation of capacitances and series resistances replacing analog compensation circuitry, wherein the computer is equipped with software for compensating capacitances and series resistances.

22. Measuring circuitry according to claim 21, wherein the I-V-converter has a further amplifier (A4) with its output being connected via a switchable capacitor (CX) to the input of the connecting amplifier (A1), the input of this amplifier being connected via a second DA-converter (DAC2) to the computer (PC).

## Patentansprüche

1. Verfahren zur Regelung und Messung von elektrischen Parametern durch eine biologische Membran (M) mit einer Messschaltung, wobei Kapazitäten und Serienwiderstände der Messschaltung (IUC) kompensiert werden, **dadurch gekennzeichnet, dass** digitale Kompensation an Stelle von analogen Schaltungen verwendet wird, wobei die digitale Kompensation durch einen Rechner (PC) durchgeführt wird, der über AD/DA-Wandler (ADC, DAC, DAC1, DAC2) mit der Messschaltung verbunden ist.

2. Verfahren nach Anspruch 1, wobei die Messschaltung ein IU-Wandler (VC) ist, der mit der biologischen Membran (M) verbunden ist, wobei ein Ausgang des IU-Wandlers zum Analysieren des I-Ausgangssignals über einen AD-Wandler (ADC) mit dem Rechner (PC) verbunden ist, wobei vorzugsweise der Rechner ein U-Eingangssignal berechnet, das über einen DA-Wandler (DAC, DAC1) in die Messschaltung eingegeben wird.

3. Verfahren nach Anspruch 2, wobei ein U-Eingangssignal aus einem aus der folgenden Gruppe von Signalen erzeugt wird: rechteckig, trapezförmig, sinusförmig.

4. Verfahren nach einem der vorhergehenden Ansprüche, wobei die Eigenschaften der Messschaltung (IUC), vorzugsweise der biologischen Membran (M) einschließlich ihrer Anschlüsse, durch den Rechner (PC) durch Verarbeiten des I-Ausgangssignals als Reaktion auf ein typisches U-Eingangssignal berechnet werden.

5. Verfahren nach einem der vorhergehenden Ansprüche, wobei ein U-Eingangssignal durch den Rechner (PC) erzeugt wird, vorzugsweise als Ergebnis des Berechnens der Eigenschaften der Messchaltung (IUC), wobei das U-Eingangssignal ein vorgegebenes I-Ausgangssignal von der Messschaltung hervorruft.

6. Verfahren nach Anspruch 5, wobei das vorgegebene I-Ausgangssignal, um einfach erkannt oder evaluiert zu werden, optimiert wird, wobei es vorzugsweise ein rechteckiges Signal ist.

7. Verfahren nach einem der vorhergehenden Ansprüche, wobei digitale Korrekturspannungssignale zum Kompensieren kapazitiver Ströme an der Membran (M) oder der Aufzeichnungskammer bzw. Halteeinrichtung in die Messschaltung (IUC) eingegeben werden, wobei die Kapazitäten vorzugsweise Streukapazitäten sind.

8. Verfahren nach Anspruch 7, wobei ein Korrekturspannungssignal durch den Rechner (PC) erzeugt und mittels einer veränderbaren oder schaltbaren Kapazität (CX) über einen DA-Wandler (DAC, DAC1) und einen Verstärker (A4) eingegeben wird, wobei es vorzugsweise zu der Elektrode eingegeben wird, die nahe der Membran (M) angeordnet ist.

9. Verfahren nach Anspruch 7, wobei ein Korrekturspannungssignal durch den Rechner (PC) erzeugt und über einen DA-Wandler (DAC, DAC1) und einen Verstärker (A4) mittels einer seriellen Kombination aus einem Widerstand und einem Kondensator (RX, CX) eingegeben wird, wobei es vorzugsweise zu der Elektrode eingegeben wird, die nahe der Membran (M) angeordnet ist.

10. Verfahren nach einem der vorhergehenden Ansprüche, wobei die elektrischen Eigenschaften einer Zellmembran oder eines Ionentransportproteins in der Membran (M) gemessen werden, wobei die Membran oder das Protein während der Berechnung der Messschaltungseigenschaften inaktiviert werden, wobei die Inaktivierung vorzugsweise durch Hyperpolarisierthalten der Membran realisiert wird.

11. Verfahren nach einem der vorhergehenden Ansprüche, wobei die elektrischen Eigenschaften einer Zellmembran oder eines Ionentransportproteins in der Membran (M) gemessen werden, wobei angenommen wird, dass die I-Ausgangsreaktion einer biologischen Membran auf eine Spannungsänderung dem Ohmschen Gesetz folgt.

12. Verfahren nach einem der vorhergehenden Ansprüche, wobei die elektrischen Eigenschaften einer Zellmembran oder eines Ionentransportproteins in der Membran (M) gemessen werden, wobei beide Membranen einer Zelle intakt sind und seriell angeordnet werden, wobei die durch die biologische Membran eingebrachte Kapazität klein ist, der Widerstand des Präparats groß ist und die digitale Kompensation in der Lage ist, Kapazitäten und Serienwiderstände in der Aufzeichnungsvorrichtung zu kompensieren.

13. Verfahren nach einem der vorhergehenden Ansprüche, wobei die elektrischen Eigenschaften einer Zellmembran oder eines Ionentransportproteins in der Membran (M) gemessen werden, wobei eine Membran einer Zelle körperlich oder elektrisch unterbrochen ist, wobei bei dieser Anordnung die durch die biologische Membran eingebrachte Kapazität groß ist, der mit der Kapazität in Serie vorgesehene Widerstand klein ist und die digitale Kompensation in der Lage ist, Kapazitäten und Serienwiderstände in der Aufzeichnungsvorrichtung zu kompensieren.

14. Verfahren nach einem der vorhergehenden Ansprüche, wobei die elektrischen Eigenschaften einer Zellmembran oder eines Ionentransportproteins in der Membran (M) gemessen werden, wobei die I-Ausgangsreaktion einer biologischen Membran auf eine Spannungsänderung antizipiert wird, indem Ionentransportproteine blockiert werden, wobei die Ionentransportproteine vorzugsweise durch Anwendung einer Lösung blockiert werden, die das Transportprotein blockiert, wobei die Lösung vorzugsweise Substanzen enthält, die den Kanal blockieren, und wobei nach der Berechnung/Kompensation der Messschaltungseigenschaften die Blockiermittel durch Lösungsaustausch entfernt werden.

15. Verfahren nach einem der vorhergehenden Ansprüche, wobei die elektrischen Eigenschaften einer Zellmembran oder eines Ionentransportproteins in der Membran (M) gemessen werden, wobei die I-Ausgangsreaktion einer biologischen Membran antizipiert wird, indem permeable Ionen von den Lösungen entfernt werden, die das Membranprotein umgeben, oder von einer Seite des Proteins entfernt werden, wobei die Spannung auf einen Wert getrieben wird, der die elektrochemische Triebkraft nahe 0 bringt.

16. Verfahren nach einem der vorhergehenden Ansprüche, wobei die elektrischen Eigenschaften einer Zellmembran oder eines Ionentransportproteins in der Membran (M) gemessen werden, wobei die I-Ausgangsreaktion einer biologischen Membran antizipiert wird, indem Cofaktoren von den Lösungen entfernt werden, die das Membranprotein umgeben, oder von einer Seite des Proteins entfernt werden, so dass ein Öffnen des Ionentransportproteins verhindert wird, beispielsweise Ca²⁺-Ionen oder andere sekundäre Botenstoffe, welche das Öffnen des Kanalproteins regeln.

17. Verfahren nach einem der vorhergehenden Ansprüche, wobei die elektrischen Eigenschaften einer Zellmembran oder eines Ionentransportproteins in der Membran (M) gemessen werden, wobei spannungsabhängige Membranproteine untersucht werden, wobei die Membranproteine zur Berechnung der Messschaltungseigenschaften durch Vermeiden von Spannungen in dem U-Eingangssignal, welche die Membranproteine aktivieren, d.h. durch Hyperpolarisierthalten der Membran oder durch Halten des U-Eingangssignals auf Pegeln, die den Kanal inaktivieren, z.B. durch Aufrechterhalten einer Depolarisierungsspannung, inaktiviert werden.

18. Verfahren nach einem der vorhergehenden Ansprüche, wobei die elektrischen Eigenschaften einer Zellmembran oder eines Ionentransportproteins in der Membran (M) gemessen werden, wobei spannungsabhängige Membranproteine untersucht werden, wobei die Membranproteine zur Berechnung der Messschaltungseigenschaften durch Auswählen von Spannungssequenzen in dem U-Eingangssignal, welche die Membranproteine inaktivieren oder aktivieren, entweder inaktiviert oder aktiviert werden.

19. Verfahren nach einem der vorhergehenden Ansprüche, wobei die elektrischen Eigenschaften einer Zellmembran oder eines Ionentransportproteins in der Membran (M) gemessen werden, wobei das U-Eingangssignal und das I-Ausgangssignal verglichen werden, um Änderungen der Membrankapazität, beispielsweise durch Änderungen der Membranfläche durch Membranfusion oder Membranrückführung, in Abhängigkeit von der Zeit zu berechnen, wobei das U-Eingangssignal vorzugsweise sinusförmig ist und die Phasenverschiebung zwischen Eingangs- und Ausgangssignal durch die Software berechnet wird.

20. Verfahren nach einem der vorhergehenden Ansprüche, wobei das U-Eingangssignal und das I-Ausgangssignal verglichen werden, um Änderungen der Membrankapazität, beispielsweise durch Änderungen der Membranfläche durch Membranfusion oder Membranrückführung, in Abhängigkeit von der Zeit zu berechnen, wobei das U-Eingangssignal vorzugsweise sinusförmig ist und die Phasenverschiebung zwischen Eingangs- und Ausgangssignal durch die Software berechnet wird.

21. Messschaltung zur Regelung und Messung von elektrischem Strom durch eine biologische Membran, umfassend eine IU-Wandlerschaltung (IUC), wobei der IU-Wandler einen Verbindungsverstärker (A1) zur Verbindung mit einer Sonde, einen Eingangsverstärker (A3) und einen Ausgangsverstärker (A2) aufweist, wobei der Eingangsverstärker und der Ausgangsverstärker über DA/AD-Wandler (DAC, ADC) mit einem Rechner (PC) verbunden sind, mit Mitteln zum Kompensieren von Kapazitäten und Serienwiderständen, **gekennzeichnet durch** eine digitale Kompensation von Kapazitäten und Serienwiderständen, welche analoge Kompensationsschaltungen ersetzt, wobei der Rechner mit Software zum Kompensieren von Kapazitäten und Serienwiderständen ausgestattet ist.

22. Messschaltung nach Anspruch 21, wobei der IU-Wandler einen weiteren Verstärker (A4) aufweist, dessen Ausgang über einen schaltbaren Kondensator (CX) mit dem Eingang des Verbindungsverstärkers (A1) verbunden ist, wobei der Eingang dieses Verstärkers über einen zweiten DA-Wandler (DAC2) mit dem Rechner (PC) verbunden ist.

## Revendications

1. Procédé de commande et de mesure de paramètres électriques à travers une membrane biologique (M) à l'aide d'une circuiterie de mesure, où des capacités et des résistances en série de la circuiterie de mesure (IUC) sont compensées, **caractérisé en ce qu'**une compensation numérique est utilisée plutôt que des circuits analogiques, où la compensation numérique est effectuée par un ordinateur (PC) relié à la circuiterie de mesure via des convertisseurs A-N/N-A (ADC, DAC, DAC1, DAC2).

2. Procédé selon la revendication 1, où la circuiterie de mesure est un convertisseur IV (VC) relié à la membrane biologique (M), une sortie du convertisseur IV étant reliée via un convertisseur A-N (ADC) à l'ordinateur (PC) pour analyser le signal de sortie I, l'ordinateur calculant de préférence un signal d'entrée V introduit via un convertisseur N-A (DAC, DAC1) à l'entrée de la circuiterie de mesure.

3. Procédé selon la revendication 2, où un signal d'entrée V est généré à partir d'un signal parmi le groupe suivant : rectangulaire, trapézoïdal, sinusoïdal.

4. Procédé selon une des revendications précédentes, où les caractéristiques de la circuiterie de mesure (IUC), de préférence de la membrane biologique (M) y compris ses connexions, sont calculées par l'ordinateur (PC) en traitant le signal de sortie I en réponse à un signal d'entrée V typique.

5. Procédé selon une des revendications précédentes, où un signal d'entrée V est généré par l'ordinateur (PC), de préférence par suite du calcul des caractéristiques de la circuiterie de mesure (IUC), le signal d'entrée V donnant un signal de sortie I prédéterminé en provenance de la circuiterie de mesure.

6. Procédé selon la revendication 5, où le signal de sortie I prédéterminé est optimisé pour être simplement détecté ou évalué, étant de préférence un signal rectangulaire.

7. Procédé selon une des revendications précédentes, où des signaux de tension de correction numérique sont introduits à l'entrée de la circuiterie de mesure (IUC) pour compenser des courants capacitifs au niveau de la membrane (M) ou du compartiment ou support d'enregistrement, respectivement, les capacités étant de préférence des capacités parasites.

8. Procédé selon la revendication 7, où un signal de tension de correction est généré par l'ordinateur (PC) et introduit via un convertisseur N-A (DAC, DAC1) et un amplificateur (A4) au moyen d'une capacité commutable ou variable (CX), étant de préférence introduit au niveau de l'électrode située près de la membrane (M).

9. Procédé selon la revendication 7, où un signal de tension de correction est généré par l'ordinateur (PC) et introduit via un convertisseur N-A (DAC, DAC1) et un amplificateur (A4) au moyen d'une combinaison en série d'une résistance et d'un condensateur (RX, CX), étant de préférence introduit au niveau de l'électrode située près de la membrane (M).

10. Procédé selon une des revendications précédentes, où les propriétés électriques d'une membrane cellulaire ou d'une protéine de transport ionique dans ladite membrane (M) sont mesurées, la membrane ou la protéine étant inactivée pendant le calcul des caractéristiques de la circuiterie de mesure, l'inactivation étant de préférence réalisée en maintenant la membrane hyperpolarisée.

11. Procédé selon une des revendications précédentes, où les propriétés électriques d'une membrane cellulaire ou d'une protéine de transport ionique dans ladite membrane (M) sont mesurées, la réponse en sortie I d'une membrane biologique à une variation de tension étant supposée suivre la loi d'Ohm.

12. Procédé selon une des revendications précédentes, où les propriétés électriques d'une membrane cellulaire ou d'une protéine de transport ionique dans ladite membrane (M) sont mesurées, les deux membranes d'une cellule étant intactes et disposées en série, la capacité introduite par la membrane biologique étant faible, la résistance de la préparation étant importante et la compensation numérique étant en mesure de compenser les capacités et les résistances en série dans l'appareil d'enregistrement.

13. Procédé selon une des revendications précédentes, où les propriétés électriques d'une membrane cellulaire ou d'une protéine de transport ionique dans ladite membrane (M) sont mesurées, une membrane d'une cellule étant physiquement ou électriquement interrompue et, dans cette disposition, la capacité introduite par la membrane biologique étant importante, la résistance en série avec la capacité étant faible et la compensation numérique étant en mesure de compenser les capacités et les résistances en série dans l'appareil d'enregistrement.

14. Procédé selon une des revendications précédentes, où les propriétés électriques d'une membrane cellulaire ou d'une protéine de transport ionique dans ladite membrane (M) sont mesurées et où la réponse en sortie I d'une membrane biologique à une variation de tension est prévue, les protéines de transport ionique étant bloquées, les protéines de transport ionique étant de préférence bloquées par application d'une solution bloquant la protéine de transport, la solution contenant de préférence des substances bloquant le canal et, à la suite du calcul / de la compensation des caractéristiques de la circuiterie de mesure, les agents bloquants étant supprimés par échange de solution.

15. Procédé selon une des revendications précédentes, où les propriétés électriques d'une membrane cellulaire ou d'une protéine de transport ionique dans ladite membrane (M) sont mesurées et où la réponse en sortie I d'une membrane biologique est prévue, les ions perméables étant supprimés des solutions entourant la protéine de la membrane ou supprimés d'un côté de la protéine, la tension étant imposée à une valeur ramenant la force motrice électrochimique au voisinage de 0.

16. Procédé selon une des revendications précédentes, où les propriétés électriques d'une membrane cellulaire ou d'une protéine de transport ionique dans ladite membrane (M) sont mesurées et où la réponse en sortie I d'une membrane biologique est prévue, les cofacteurs étant supprimés des solutions entourant la protéine de la membrane ou supprimés d'un côté de la protéine de telle sorte que l'ouverture de la protéine de transport ionique soit empêchée, par exemple des ions Ca²⁺ ou d'autres seconds messagers régulant l'ouverture de la protéine-canal.

17. Procédé selon une des revendications précédentes, où les propriétés électriques d'une membrane cellulaire ou d'une protéine de transport ionique dans ladite membrane (M) sont mesurées, des protéines de membrane dépendantes de la tension étant examinées, les protéines de membrane étant inactivées pour le calcul des caractéristiques de la circuiterie de mesure en évitant les tensions dans le signal d'entrée V qui activent les protéines de membrane, par ex. en maintenant la membrane hyperpolarisée, ou en gardant le signal d'entrée V à des niveaux qui inactivent le canal, par ex. maintenant une tension dépolarisante.

18. Procédé selon une des revendications précédentes, où les propriétés électriques d'une membrane cellulaire ou d'une protéine de transport ionique dans ladite membrane (M) sont mesurées, des protéines de membrane dépendantes de la tension étant examinées, les protéines de membrane étant soit inactivées soit activées pour le calcul des caractéristiques de la circuiterie de mesure en choisissant des suites de tensions dans le signal d'entrée V qui inactivent ou activent les protéines de membrane.

19. Procédé selon une des revendications précédentes, où les propriétés électriques d'une membrane cellulaire ou d'une protéine de transport ionique dans ladite membrane (M) sont mesurées, le signal d'entrée V et le signal de sortie I étant comparés afin de calculer des variations dans le temps de la capacité de la membrane, par exemple par des variations de l'aire de la membrane par fusion de la membrane ou de récupération de la membrane, le signal d'entrée V étant de préférence sinusoïdal et le déphasage entre les signaux d'entrée et de sortie étant calculé par le logiciel.

20. Procédé selon une des revendications précédentes, où le signal d'entrée V et le signal de sortie I sont comparés afin de calculer des variations dans le temps de la capacité de la membrane, par exemple par des variations de l'aire de la membrane par fusion de la membrane ou de récupération de la membrane. De préférence, le signal d'entrée V est sinusoïdal et le déphasage entre les signaux d'entrée et de sortie est calculé par le logiciel.

21. Circuiterie de mesure pour la commande et la mesure du courant électrique à travers une membrane biologique, comportant une circuiterie de convertisseur IV (IUC), le convertisseur IV étant muni d'un amplificateur de connexion (A1) destiné à la connexion avec une sonde, un amplificateur d'entrée (A3) et un amplificateur de sortie (A2), l'amplificateur d'entrée et l'amplificateur de sortie étant reliés via des convertisseurs N-A/A-N (DAC, ADC) à un ordinateur (PC), avec des moyens de compenser les capacités et les résistances en série, **caractérisée par** le remplacement de la circuiterie de compensation analogique par une compensation numérique des capacités et des résistances en série, l'ordinateur étant équipé d'un logiciel pour compenser les capacités et les résistances en série.

22. Procédé de mesure selon la revendication 21, le convertisseur IV étant muni d'un amplificateur supplémentaire (A4) dont la sortie est reliée à l'entrée de l'amplificateur de connexion (A1) via une capacité commutable (CX), l'entrée de cet amplificateur étant reliée à l'ordinateur (PC) via un deuxième convertisseur N-A (DAC2).
